# EUROPÄISCHE PATENTSCHRIFT

(11) **EP 1 626 664 B1**
(45) Veröffentlichungstag und Bekanntmachung des Hinweises auf die Patenterteilung: **25.08.2010**
(21) Anmeldenummer: 04739500.9
(22) Anmeldetag: 28.05.2004
(51) Int. Cl.: A61B 17/14, B23D 51/10

(54) **CHIRURGISCHES SÄGEBLATT**
SURGICAL SAW-BLADE
LAME DE SCIE CHIRURGICALE

(30) Priorität: 28.05.2003 DE 10325391
(43) Veröffentlichungstag der Anmeldung: 22.02.2006
(60) Teilanmeldung: 07117713.3
(73) Patentinhaber: Aesculap AG, 78532 Tuttlingen (DE)
(72) Erfinder: BLUST, Edgar, 78126 Königsfeld (DE); BUTSCH, Werner, 78589 Dürbheim (DE); GASSNER, Stefan, 78194 Immendingen-Hattingen (DE); HAGEN, Thomas, 78532 Tuttlingen (DE); HÄUSLER, Rainer, 78532 Tuttlingen (DE); HÖGERLE, Alois, 78532 Tuttlingen (DE); KLEINWÄCHTER, Timo, 78532 Tuttlingen (DE)
(74) Vertreter: Regelmann, Thomas
(86) Internationale Anmeldenummer: PCT/EP2004/005912
(87) Internationale Veröffentlichungsnummer: WO 2004/105623

(56) Entgegenhaltungen:
- DE-A- 2 935 732
- US-A- 5 735 866
- US-A- 5 846 244

## Beschreibung

Die Erfindung betrifft ein chirurgisches Sägeblatt mit einem Haltekörper und einer Sägeeinrichtung, welche an dem Haltekörper gehalten ist und welche relativ zu dem Haltekörper beweglich ist, wobei die Sägeeinrichtung durch ein Sägeband als Endloseinrichtung gebildet ist, welches an einem Seitenbereich des Haltekörpers geführt ist.

Chirurgische Sägeblätter sind beispielsweise aus der DE 101 00 630 C1 und der DE 202 11 397 U1 bekannt.

Aus der DE 26 11 720 A1 ist ein Bandsägen-Skalpell bekannt. Die DE 27 58 380 A1 offenbart eine Kette für ein motorisch angetriebenes Trenngerät für den medizinischen Bereich.

Die WO 99/20184 A1 und die US 5,846,244 offenbaren chirurgische Sägen.

Aus der US 5,735,866 ist ein Sägenblatt bekannt, welches zwei Bereiche aufweist, die einstellbar relativ zueinander positionierbar sind, um die Gesamtlänge des Sägenblatts einstellen zu können.

Aus der DE 29 35 732 A1 (Basis für den Oberbegriff des Anspruchs 1) ist eine Vorrichtung zum Antrieb einer Kette eines Präzisions-Skalpells mit einem Kettenrad zum Antrieb und zur Umlenkung der Kette bekannt.

Chirurgische Sägeblätter werden insbesondere im Zusammenhang mit Oszillationssägen für orthopädische Zwecke eingesetzt, beispielsweise um Platz zu schaffen für eine Knieendoprothese.

Der Erfindung liegt die Aufgabe zugrunde, ein chirurgisches Sägeblatt der eingangs genannten Art zu schaffen, welches für verbesserte Handhabungseigenschaften sorgt.

Diese Aufgabe wird bei dem eingangs genannten chirurgischen Sägeblatt erfindungsgemäß dadurch gelöst, daß das Sägeband einen elliptischen Querschnitt aufweist, wobei die Durchmesserlänge an der großen Halbachse größer ist als die Dicke des Haltekörpers.

Dadurch, daß die Sägeeinrichtung relativ zu dem Haltekörper beweglich ist, um so eine Sägebewegung durchzuführen, ist die bewegte Masse geringer, als wenn der gesamte Haltekörper bewegt wird. Damit läßt sich die Belastung eines Getriebes einer Antriebssäge verringern und dadurch wiederum läßt sich die Lebensdauer des Sägengetriebes erhöhen. Chirurgische Sägen werden üblicherweise im Akkubetrieb eingesetzt. Durch die erfindungsgemäße Lösung wird die bewegte Masse reduziert, so daß sich durch den verringerten Energiebedarf auch die Sägenlaufzeit erhöhen läßt.

Die Sägeeinrichtung ist durch eine Endloseinrichtung gebildet, welche an einem Seitenbereich des Haltekörpers geführt ist. Eine solche Führung läßt sich auf einfache Weise beispielsweise über eine Nut ausbilden, in welcher die Endloseinrichtung geführt ist.

Die Endloseinrichtung ist ein Sägeband (Sägedraht). Über ein solches Sägeband läßt sich bei entsprechender Ausbildung ein Sägeschnitt einbringen.

Ganz besonders vorteilhaft ist es dann, wenn das Sägeblatt im Querschnitt in senkrecht aufeinander stehenden Richtungen unterschiedliche Durchmesserlängen aufweist. Das Sägeblatt weist einen elliptischen Querschnitt auf, wobei die Durchmesserlänge an der großen Halbachse größer ist als die Dicke des Haltekörpers. Es läßt sich dadurch erreichen, daß über das Sägeband ein Sägeschnitt einbringbar ist, welcher eine größere Breite quer zur Bewegungsrichtung der Sägeneinrichtung aufweist, als es der Dicke des Haltekörpers entspricht. Dadurch ist gewährleistet, daß sich Sägespäne auf einfache Weise aus dem Sägespalt abführen lassen, da Abführwege durch den Haltekörper nicht blockiert werden. Weiterhin ist die Aufheizung des Gewebes um den Sägespalt minimiert, da der Reibungskontakt des Haltekörpers mit dem Gewebe minimiert ist.

Insbesondere ist die Sägeeinrichtung angetrieben, um so einen Sägeschnitt einbringen zu können.

Ein Antrieb und insbesondere ein Antriebsmotor zur Bewegung der Sägeeinrichtung ist dabei vorzugsweise extern angeordnet, das heißt in einer Säge angeordnet, an welche das Sägeblatt gekoppelt ist.

Der Antrieb kann grundsätzlich bei entsprechender Ausgestaltung der Sägeeinrichtung direkt an diese gekoppelt sein. Es ist vorteilhaft, wenn die Sägeeinrichtung über den Haltekörper an einen Antrieb zur Bewegung der Sägeeinrichtung gekoppelt ist. Dadurch läßt sich die Handhabung vereinfachen, wenn insbesondere vorgesehen ist, daß durch die Fixierung des Haltekörpers an der Säge gleichzeitig eine Ankopplung des Antriebs erfolgt. Es läßt sich so auch erreichen, daß Kraftübertragungselemente (Zwischengetriebe), die benötigt werden, um den Antrieb der Säge an die Sägeeinrichtung zu koppeln, verdeckt in dem Haltekörper unterbringbar sind, so daß insbesondere die Verunreinigungsanfälligkeit verringert ist und auch ein Bediener geschützt ist.

Vorzugsweise sind an dem Haltekörper Kraftübertragungselemente zur Bewegung der Sägeeinrichtung angeordnet. Dadurch läßt sich das Drehmoment des Antriebs der Säge in einer räumlichen Distanz zu der Sägeeinrichtung in den Haltekörper einkoppeln.

Besonders günstig ist es, wenn die Kraftübertragungselemente im Haltekörper angeordnet sind. Dadurch sind sie vom Außenraum geschützt, das heißt besser gegen Verunreinigungen geschützt.

Insbesondere sind die Kraftübertragungselemente zwischen gegenüberliegenden äußeren Oberflächen des Haltekörpers angeordnet, so daß sie gegenüber dem Außenraum verdeckt angeordnet sind.

Weiterhin ist es günstig, wenn der Haltekörper einen Kopplungsbereich umfaßt, über den ein externer Antrieb wirksam an Kraftübertragungselemente zur Bewegung der Sägeeinrichtung koppelbar ist.

In einer einfachen Ausführungsform umfaßt der Kopplungsbereich eine Öffnung in dem Haltekörper. Durch dieses Öffnung hindurch kann dann ein Kopplungselement der Säge an Kraftübertragungselemente des Sägeblattes angreifen.

Zur Einbringung eines Sägeschnittes ist es günstig, wenn der Haltekörper an einem Sägeende eine größere Breite aufweist als an einem gegenüberliegenden Ende. Es läßt sich dadurch ein langer Sägeschnitt einbringen, wobei andererseits die Oberfläche des Haltekörpers, mit welcher diese in einen Sägespalt eintaucht, minimiert ist, da von dem Sägeende weg die Breite des Haltekörpers abnimmt. Dadurch wiederum läßt sich sicherstellen, daß Sägespäne aus dem Sägespalt abführbar sind.

Es ist vorgesehen, daß sich die Sägeeinrichtung an einem Sägeende des Haltekörpers quer zu einer Längsrichtung des Haltekörpers bewegt. Eine solche Bewegungsform läßt sich auf einfache Weise erreichen, wobei sich lange Sägeschnitte einbringen lassen.

Es kann vorgesehen sein, daß die Sägeeinrichtung eine oszillierende Bewegung durchführt.

Es kann auch vorgesehen sein, daß eine Sägeeinrichtung eine umlaufende Bewegung durchführt.

Bei einer umlaufenden Bewegung kann es sich um eine um den Haltekörper umlaufende Bewegung handeln, indem beispielsweise eine Endloskette oder ein Endlosband um den Haltekörper geführt wird.

Bei der umlaufenden Bewegung kann es sich auch um eine Rotationsbewegung um eine Drehachse handeln, beispielsweise in der Art der Rotation einer Kreissäge.

Es ist dann weiterhin günstig, wenn eine Umlenkeinrichtung für das Sägeband vorgesehen ist, um das Sägeband an dem Sägeende so an dem Haltekörper auszurichten, daß ein Schnittspalt mit einer Höhe größer als die Dicke des Haltekörpers erzeugbar ist. Zur Führung des Sägebandes an dem Haltekörper sollte günstigerweise das Sägeband eine Durchmesserlänge aufweisen, welche kleiner ist als die Dicke des Haltekörpers. Durch die Umlenkeinrichtung läßt sich über große Teile des Haltekörpers eine solche Führung ausbilden, wobei andererseits gewährleistet ist, daß sich ein Schnittspalt erzeugen läßt, welcher eine größere Höhe aufweist als es der Dicke des Haltekörpers entspricht.

Die nachfolgende Beschreibung bevorzugter Ausführungsformen dient im Zusammenhang mit der Zeichnung der näheren Erläuterung der Erfindung. Es zeigen:
- Figur 1: eine Draufsicht auf ein erstes Ausführungsbeispiel eines erfin- dungsgemäßen chirurgischen Sägeblattes; und
- Figur 2: eine Schnittansicht des chirurgischen Sägeblattes gemäß Figur 1 längs der Linie 2-2.

Chirurgische Sägeblätter werden insbesondere für orthopädische Zwecke im Zusammenhang mit akkubetriebenen Sägen und insbesondere Oszillationssägen eingesetzt. Beispielsweise muß beim Einsatz von Knieendoprothesen zuvor Platz für die Prothese geschaffen werden.

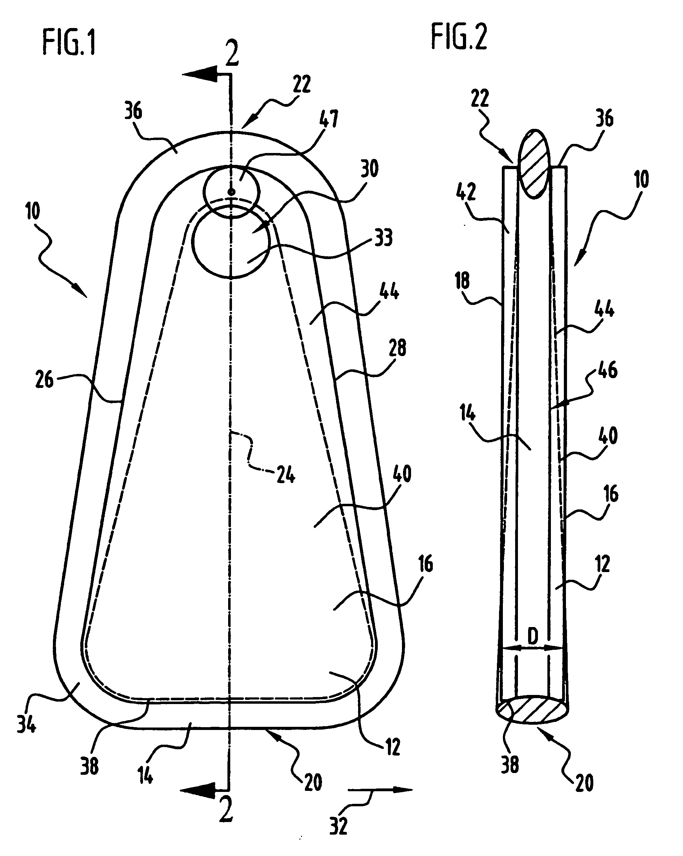

Ein erstes Ausführungsbeispiel eines erfindungsgemäßen chirurgischen Sägeblattes zum Einsatz mit einer Säge, welches in den Figuren 1 und 2 gezeigt und dort als Ganzes mit 10 bezeichnet ist, umfaßt einen Haltekörper 12, an welchem eine Sägeeinrichtung 14 gehalten ist. Über den Haltekörper 12 ist das Sägeblatt 10 an der Säge fixierbar, wobei die Sägebewegung des Sägeblattes 10 durch einen Antrieb der Säge angetrieben ist.

Der Haltekörper 12 hat gegenüberliegende im wesentlichen parallele Oberflächen 16, 18 zwischen welchen der Haltekörper 12 gebildet ist. Die Oberflächen 16, 18 sind im wesentlichen eben, wobei eine Oberflächenstruktur vorgesehen sein kann, welche konvex gekrümmte Ausnehmungen aufweist. Eine solche Oberflächenstruktur ist in der DE 101 00 630 C1 beschrieben.

Der Haltekörper 12 weist ein Sägeende 20 und ein gegenüberliegendes Ende 22 auf. An dem Sägeende 20 ist der Haltekörper 12 breiter als an dem gegenüberliegenden Ende 22, so daß bezogen auf eine Längsachse 24 des Haltekörpers 12 gegenüberliegende Seiten 26, 28 des Haltekörpers 12 in einem spitzen Winkel von dem Ende 22 zu dem Sägeende 20 orientiert sind.

Der Haltekörper 12 weist einen Kopplungsbereich 30 (Anschluß) für die Säge auf, über welchen eben das Sägeblatt 10 an der Säge fixierbar ist. Der Kopplungsbereich umfaßt vorzugsweise eine Öffnung 33.

Die Sägeeinrichtung 14 ist relativ zu dem Haltekörper 12 beweglich, wobei eine Bewegungsrichtung 32 der Sägeeinrichtung 14 im Bereich des Sägeendes 20 quer zur Oberflächennormale der Oberflächen 16, 18 ist und quer zur Längsachse 24 und insbesondere rechtwinklig zu dieser ist.

Die Sägeeinrichtung 14 ist bei dem Sägeblatt 10 als Sägeband 34 (Sägedraht) ausgebildet. Dieses Sägeband 34 ist umlaufend um den Haltekörper 12 geführt. Dazu sind die Seiten 26, 28 und die Stirnseite 36 des Haltekörpers 12 zumindest teilweise als Führungen ausgebildet. Das Ende 22 liegt dabei an der Stirnseite 36 und das Sägeende 20 an einer der Stirnseite 36 gegenüberliegenden Stirnseite 38.

Es kann beispielsweise vorgesehen sein, daß randseitig zwischen Deckelelementen 40 und 42, an welchen jeweils die Oberflächen 16 und 18 gebildet sind, eine zwischen den Seiten 26, 28 und 36 umlaufende Nut 44 angeordnet ist, in welcher das Sägeband 34 geführt ist.

Das Sägeband 34 ist eine Endloseinrichtung, das heißt ein Endlosband, welches an einem durch die Nut 44 gebildeten Seitenbereich des Haltekörpers 12 geführt ist.

Es ist vorgesehen, daß das Sägeband 34 in einem Querschnitt in senkrecht aufeinander stehenden Richtungen unterschiedliche Durchmesserlängen aufweist, das heißt in einem solchen Querschnitt unterschiedliche Breiten aufweist. Das Sägeband 34 weist einen elliptischen Querschnitt auf. An der großen Halbachse ist dann die Durchmesserlänge größer als an der kleinen Halbachse. Das Sägeband 34 hat dabei solche Abmessungen, daß die Durchmesserlänge an der großen Halbachse größer ist als eine Dicke D des Haltekörpers 12 zwischen den Oberflächen 16 und 18, und daß die Durchmesserlänge an der kleinen Halbachse kleiner ist als die Dicke D und insbesondere höchstens der (maximalen) Ausdehnung der Nut 44 entspricht.

An dem Sägeende 20 ist das Sägeband 34 so aufgestellt, daß seine Höhenabmessung in der Dickenrichtung des Haltekörpers 12 größer ist als die Dicke D. Dadurch läßt sich ein Schnittspalt erzeugen, der breiter ist als die Dicke D.

An dem Haltekörper 12 und insbesondere den Seiten 26, 28 ist eine Umlenkeinrichtung 46 angeordnet, um eben das Sägeband 34 so umzulenken, daß der größere Durchmesser des Sägebandes 34 am Sägeende 20 parallel zu diesem Sägeende 20 ausgerichtet ist. Bezogen auf die Bewegungsrichtung 32 ist dabei beispielsweise an der Seite 26 die Umlenkeinrichtung 46 so ausgebildet, daß sie das Sägeband 34 für das Sägeende 20 aufrichtet, während die Umlenkeinrichtung 46 an der gegenüberliegenden Seite 28 so ausgebildet ist, daß sie das Sägeband 24 wieder umlegt, um dieses in der Nut 44 führen zu können.

Das Sägeband 34 läuft in der Nut 44 um den Seitenumfang des Haltekörpers 12 um. Diese Bewegung des Sägebandes 34 ist durch die Säge angetrieben. Ein Antrieb der Säge kann dabei direkt auf das Sägeband 34 wirken oder es können an dem Haltekörper 12 Kraftübertragungselemente wie Reibräder 47 vorgesehen sein, auf welche der Antrieb wirkt und über die die Bewegung des Sägebandes 34 in der Bewegungsrichtung 32 antreibbar ist. Der Sägenantrieb kann über die Öffnung 33 auf ein Reibrad 47 wirken und dies antreiben, wobei das Reibrad wiederum auf das Sägeband 34 wirkt und dessen Bewegung antreibt. Die Kraftübertragungselemente des Haltekörpers 12 sind dann insbesondere verdeckt zwischen den Deckelementen 40, 42 angeordnet und über die Öffnung 33 kann der Antrieb der Säge an diese Kraftübertragungselemente gekoppelt werden (in den Figuren 1 und 2 nicht gezeigt).

Zur Einbringung eines Sägeschnitts wird das Sägeband 34 als Sägeeinrichtung 14 relativ zu dem Haltekörper 12 in einer umlaufenden Bewegung um einen umlaufenden Seitenrand des Haltekörpers 12 bewegt. Da nur das Sägeband 34 bewegt wird und nicht der Haltekörper 12, entspricht die bewegte Masse der Masse des Sägebandes 34. Diese Masse ist im Vergleich zu der des Haltekörpers 12 gering. Dadurch läßt sich die Belastung des Getriebes der Säge verringern und die Laufzeit der Säge läßt sich erhöhen (üblicherweise werden chirurgische Sägen über Akkus betrieben).

Statt einer umlaufenden Bewegung des Sägebandes 34 kann auch eine oszillierende Bewegung vorgesehen sein, das heißt das Sägeband 34 bewegt sich am Sägeende 20 oszillierend in der Bewegungsrichtung 32 und in die Gegenrichtung.

## Patentansprüche

1. Chirurgisches Sägeblatt mit einem Haltekörper (12) und einer Sägeeinrichtung (34), welche an dem Haltekörper (12) gehalten ist und weiche relativ zu dem Haltekörper (12) beweglich ist, wobei die Sägeeinrichtung durch eine Endloseinrichtung (34; 56) gebildet ist, welche an einem Seitenbereich (44; 52) des Haltekörpers (12; 50) geführt ist,
**dadurch gekennzeichnet , daß** die Endloseinrichtung ein Sägeband (34) ist, das einen elliptischen Querschnitt aufweist, wobei die Durchmesserlänge an der großen Halbachse größer ist als die Dicke (D) des Haltekörpers (12).

2. Chirurgisches Sägeblatt nach Anspruch 1, **dadurch gekennzeichnet, daß** die Sägeeinrichtung (34) angetrieben ist.

3. Chirurgisches Sägeblatt nach Anspruch 2, **dadurch gekennzeichnet, daß** ein Antrieb zur Bewegung der Sägeeinrichtung (34) extern angeordnet ist.

4. Chirurgisches Sägeblatt nach Anspruch 2 oder 3, **dadurch gekennzeichnet, daß** die Sägeeinrichtung (34) über den Haltekörper (12) an einen Antrieb zur Bewegung der Sägeeinrichtung (34) gekoppelt ist.

5. Chirurgisches Sägeblatt nach einem der vorangehenden Ansprüche, **dadurch gekennzeichnet, daß** an dem Haltekörper Kraftübertragungselemente zur Bewegung der Sägeeinrichtung angeordnet sind.

6. Chirurgisches Sägeblatt nach Anspruch 5, **dadurch gekennzeichnet, daß** die Kraftübertragungselemente im Haltekörper angeordnet sind.

7. Chirurgisches Sägeblatt nach Anspruch 5 oder 6, **dadurch gekennzeichnet, daß** die Kraftübertragungselemente zwischen gegenüberliegenden äußeren Oberflächen (16, 18) des Haltekörpers angeordnet sind.

8. Chirurgisches Sägeblatt nach einem der Ansprüche 5 bis 7, **dadurch gekennzeichnet, daß** der Haltekörper (12) einen Kopplungsbereich (30) umfaßt, über den ein externer Antrieb wirksam an Kraftübertragungselemente zur Bewegung der Sägeeinrichtung (34) koppelbar ist.

9. Chirurgisches Sägeblatt nach Anspruch 8, **dadurch gekennzeichnet, daß** der Kopplungsbereich (30) eine Öffnung (33) an dem Haltekörper (12) umfaßt.

10. Chirurgisches Sägeblatt nach einem der vorangehenden Ansprüche, **dadurch gekennzeichnet, daß** der Haltekörper (12) an einem Sägeende (20) eine größere Breite aufweist als an einem gegenüberliegenden Ende (22).

11. Chirurgisches Sägeblatt nach einem der vorangehenden Ansprüche, **dadurch gekennzeichnet, daß** sich die Sägeeinrichtung (34) an einem Sägeende (20) des Haltekörpers (12) quer zu einer Längsrichtung (24) des Haltekörpers (12) bewegt.

12. Chirurgisches Sägeblatt nach einem der vorangehenden Ansprüche, **dadurch gekennzeichnet, daß** die Sägeeinrichtung (34) eine oszillierende Bewegung durchführt.

13. Chirurgisches Sägeblatt nach einem der Ansprüche 1 bis 11, **dadurch gekennzeichnet, daß** die Sägeeinrichtung eine umlaufende Bewegung durchführt.

14. Chirurgisches Sägeblatt nach Anspruch 13, **dadurch gekennzeichnet, daß** die Sägeeinrichtung eine um den Haltekörper (12) umlaufende Bewegung durchführt.

15. Chirurgisches Sägeblatt nach einem der vorangehenden Ansprüche,
**dadurch gekennzeichnet, daß** das Sägeband (34) im Querschnitt in senkrecht aufeinander stehenden Richtungen unterschiedliche Durchmesserlängen aufweist.

16. Chirurgisches Sägeblatt nach einem der vorangehenden Ansprüche,
**dadurch gekennzeichnet, daß** eine Umlenkeinrichtung (46) für das Sägeband (34) vorgesehen ist, um das Sägeband (34) an dem Sägeende (20) so an dem Haltekörper (12) auszurichten, daß ein Schnittspalt mit einer Breite größer als die Dicke (D) des Haltekörpers (12 ) erzeugbar ist.

## Claims

1. A surgical saw blade having a holding body (12) and a sawing device (34) which is held on the holding body (12) and is movable relative to the holding body (12), wherein the sawing device is formed by an endless device (34; 56) guided on a lateral region (44; 52) of the holding body (12; 50), **characterised in that** the endless device is a saw band (34) with an elliptical cross-section, wherein the diameter length at the large semi axis is greater than the thickness (D) of the holding body (12).

2. A surgical saw blade according to claim 1, **characterised in that** the sawing device (34) is driven.

3. A surgical saw blade according to claim 2, **characterised in that** a drive for movement of the sawing device (34) is arranged externally.

4. A surgical saw blade according to claim 2 or 3, **characterised in that** the sawing device (34) is coupled, via the holding body (12), to a drive for movement of the sawing device (34).

5. A surgical saw blade according to any one of the preceding claims, **characterised in that** force transmission elements for movement of the sawing device are arranged on the holding body.

6. A surgical saw blade according to claim 5, **characterised in that** the force transmission elements are arranged in the holding body.

7. A surgical saw blade according to claim 5 or 6, **characterised in that** the force transmission elements are arranged between facing, outer surfaces (16, 18) of the holding body.

8. A surgical saw blade according to any one of claims 5 to 7, **characterised in that** the holding body (12) comprises a coupling region (30) via which an external drive can be operatively coupled to force transmission elements for movement of the sawing device (34).

9. A surgical saw blade according to claim 8, **characterised in that** the coupling region (30) comprises an opening (33) at the holding body (12).

10. A surgical saw blade according to any one of the preceding claims, **characterised in that** the holding body (12) is greater in width at one sawing end (20) than at an opposite end (22).

11. A surgical saw blade according to any one of the preceding claims, **characterised in that** at one sawing end (20) of the holding body (12), the sawing device (34) moves transversely to a longitudinal direction (24) of the holding body (12).

12. A surgical saw blade according to any one of the preceding claims, **characterised in that** the sawing device (34) executes an oscillating motion.

13. A surgical saw blade according to any one of claims 1 to 11, **characterised in that** the sawing device executes a circulating motion.

14. A surgical saw blade according to claim 13, **characterised in that** the sawing device executes a circulating motion about the holding body (12).

15. A surgical saw blade according to any one of the preceding claims, **characterised in that** in cross-section and in directions perpendicular to one another, the saw band (34) has different diameter lengths.

16. A surgical saw blade according to any one of the preceding claims, **characterised in that** a deflection device (46) for the saw band (34) is provided, in order for the saw band (34) at the sawing end (20) to be oriented in such a manner on the holding body (12) that a kerf with a width greater than the thickness (D) of the holding body (12) can be produced.

## Revendications

1. Lame de scie chirurgicale comprenant un corps de support (12) et un dispositif de sciage (34), qui est supporté ou maintenu par le corps de support (12) et est mobile par rapport au corps de support (12), le dispositif de sciage étant formé par un dispositif sans fin (34 ; 56), qui est guidé dans une zone latéral (44 ; 52) du corps de support (12 ; 50), **caractérisée en ce que** le dispositif sans fin est une bande ou un ruban de scie (34), qui présente une section transversale elliptique, la langueur de diamètre au biveau du grand demi-axe étant supérieure à l'épaisseur (D) du corps de support (12).

2. Lame de scie chirurgicale selon la revendication 1, **caractérisée en ce que** le dispositif de sciage (34) est entraîné.

3. Lame de scie chirurgicale selon la revendication 2, **caractérisée en ce qu'**un système d'entraînement pour assurer le mouvement du dispositif de sciage (34), est agencé de manière externe.

4. Lame de scie chirurgicale selon la revendication 2 ou la revendication 3, **caractérisée en ce que** le dispositif de sciage (34) est couplé, par l'intermédiaire du corps de support (12), à un système d'entraînement pour assurer le mouvement du dispositif de sciage (34).

5. Lame de scie chirurgicale selon l'une des revendications précédentes, **caractérisée en ce que** sur le corps de support sont agencés des éléments de transmission de force pour assurer le mouvement du dispositif de sciage.

6. Lame de scie chirurgicale selon la revendication 5, **caractérisée en ce que** les éléments de transmission de force sont agencées dans le corps de support.

7. Lame de scie chirurgicale selon la revendication 5 ou la revendication 6, **caractérisée en ce que** les éléments de transmission de force sont agencés entre deux surfaces extérieures opposées (16, 18) du corps de support .

8. Lame de scie chirurgicale selon l'une des revendications 5 à 7, **caractérisée en ce que** le corps de support (12) comprend une zone de couplage (30) par l'intermédiaire de laquelle un système d'entraînement extérieur peut être couplé de manière active à des éléments de transmission de force pour assurer le mouvement du dispositif de sciage (34).

9. Lame de scie chirurgicale selon la revendication 8, **caractérisée en ce que** la zone de couplage (30) comprend une ouverture (33) sur le corps de support (12).

10. Lame de scie chirurgicale selon l'une des revendications précédentes, **caractérisée en ce que** le corps de support (12) présente, à une extrémité de sciage (20), une largeur plus grande qu' à une extrémité opposée (22).

11. Lame de scie chirurgicale selon l'une des revendications précédentes, **caractérisée en ce qu'**à une extrémité de sciage (20) du corps de support (12), le mouvement du dispositif de sciage (34) s'effectue transversalement à une direction longitudinale (24) du corps de support (12).

12. Lame de scie chirurgicale selon l'une des revendications précédentes, **caractérisée en ce que** le dispositif de sciage (34) effectue un mouvement oscillant.

13. Lame de scie chirurgicale selon l'une des revendications 1 à 11, **caractérisée en ce que** le dispositif de sciage effectue un mouvement de révolution.

14. Lame de scie chirurgicale selon la revendication 13, **caractérisée en ce que** le dispositif de sciage effectue un mouvement de révolution autour du corps de support (12).

15. Lame de scie chirurgicale selon l'une des revendications précédentes, **caractérisée en ce que** la bande ou le ruban de sciage (34) présente, en section transversale, dans des directions perpendiculaires l'une à l'autre, des longueurs de diamètre différentes.

16. Lame de scie chirurgicale selon l'une des revendications précédentes, **caractérisée en ce qu'**il est prévu un dispositif de déviation (46) pour la bande ou le ruban de sciage (34), en vue d'orienter la bande ou le ruban de sciage (34) sur le corps de support (12), à l'extrémité de sciage (20), de façon à permettre d'engendrer un interstice de coupe d'une largeur supérieure à l'épaisseur (D) du corps de support (12).
